(19) Europäisches Patentamt
European Patent Office
Office européen des brevets

(11) EP 0 414 632 B1

(12) **EUROPEAN PATENT SPECIFICATION**

(45) Date of publication and mention of the grant of the patent:
**23.10.1996 Bulletin 1996/43**

(51) Int Cl.[6]: **C07K 7/64**, A61K 38/13

(21) Application number: **90810567.9**

(22) Date of filing: **23.07.1990**

(54) **Cyclosporin derivatives**

Cyclosporin- Derivate

Dérivés de cyclosporine

(84) Designated Contracting States:
**AT BE CH DE DK ES FR GB GR IT LI LU NL SE**

(30) Priority: **24.07.1989 GB 8916901**

(43) Date of publication of application:
**27.02.1991 Bulletin 1991/09**

(73) Proprietors:
- **SANDOZ LTD.**
  **4002 Basel (CH)**
  Designated Contracting States:
  **BE CH DK ES FR GB GR IT LI LU NL SE**
- **SANDOZ-PATENT-GMBH**
  **79539 Lörrach (DE)**
  Designated Contracting States:
  **DE**
- **SANDOZ-ERFINDUNGEN Verwaltungsgesellschaft m.b.H.**
  **1235 Wien (AT)**
  Designated Contracting States:
  **AT**

(72) Inventor: **Eberle, Marcel Karl**
**CH-4125 Riehen (CH)**

(56) References cited:
**EP-A- 0 056 782** **GB-A- 2 155 936**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 414 632 B1

## Description

The present invention relates to novel cyclosporins, their use as pharmaceuticals and pharmaceutical compositions comprising them, as well as to processes for their production.

The cyclosporins comprise a class of structurally distinctive, cyclic, poly-N-methylated undecapeptides, commonly possessing pharmacological, in particular immunosuppressive, anti-inflammatory and/or antiparasitic activity. The first of the cyclosporins to be isolated was the naturally occurring fungal metabolite Ciclosporin or Cyclosporine, also known as cyclosporin A and commercially available under the Registered Trademark SANDIMMUN$^R$ or SANDIMMUNE$^R$. Ciclosporin is the cyclosporin of formula A.

MeBmt-αAbu-Sar-MeLeu-Val-MeLeu-Ala-(D)Ala-MeLeu-MeLeu-MeVal
1 2 3 4 5 6 7 8 9 10 11 (A)

where MeBmt represents the N-methyl-(4R)-4-but-2E-en-1-yl-4-methyl--(L)threonyl residue of formula B

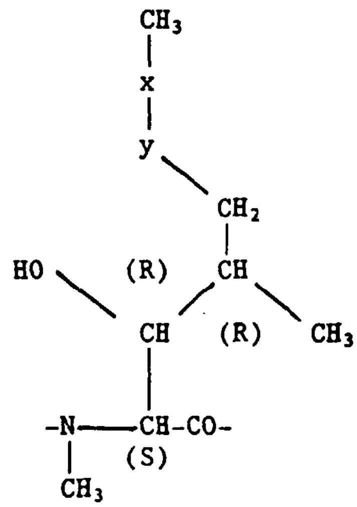

(B)

in which -x-y- is -CH=CH- (trans).

Since the original discovery of Ciclosporin, a wide variety of naturally occurring cyclosporins have been isolated and identified and many further non-natural cyclosporins have been prepared by total- or semi-synthetic means or by the application of modified culture techniques. The class comprised by the cyclosporins is thus now substantial and includes, for example, the naturally occurring cyclosporins A through Z [cf. Traber et al; 1, Helv. Chim. Acta, 60, 1247-1255 (1977); Traber et al; 2, Helv. Chim. Acta, 65, 1655-1667 (1982); Kobel et al, Europ. J. Applied Microbiology and Biotechnology, 14, 273-240 1982); and von Wartburg et al, Progress in Allergy, 38, 28-45, 1986)], as well as various non-natural cyclosporin derivatives and artificial or synthetic cyclosporins including dihydro-cyclosporins [in which the moiety -x-y- of the MeBmt residue (formula B above) is saturated to give -x-y- = $-CH_2-CH_2-$]; derivatised cyclosporins (e.g. in which the 3′-O-atom of the MeBmt residue is acylated or a further substituent is introduced at the α-carbon atom of the sarcosyl residue at the 3-position); cyclosporins in which the MeBmt residue is present in isomeric form (e.g. in which the configuration across positions 6' and 7' of the MeBmt residue is cis rather than trans); and cyclosporins in which variant amino acids are incorporated at specific positions within the peptide sequence, e.g. employing the total synthetic method for the production of cyclosporins developed by R. Wenger - see e.g. Traber et al. 1, Traber et al, 2 and Kobel et al., loc. cit.; US Patents Nos 4,108,985, 4,220,641, 4,288,431, 4,554,351, 4,396,542 and 4,798,823 European Patent Publications Nos. 34,567A, 56,782A, 300,784A and 300,785A; International Patent Publication No WO 86/02080 and UK Patent Publications Nos, 2,206,119 and 2,207,678; Wenger 1, Transpl. Proc., 15 Suppl. 1:2230 (1983); Wenger 2., Angew. Chem. Int. Ed. 24 77 (1985) and Wenger 3., Progress in the Chemistry of Organic Natural Products, 50, 123 (1986).

Uk Patent Publication 2155936 discloses [O-acetyl-(D)-Ser]$^8$ Cyclosporine.

The class comprised by the cyclosporins is thus now very large indeed and includes, for example, [Thr]$^2$-, [Val]$^2$-,

[Nva]$^2$- and [Nva]$^2$-[Nva]$^5$-Ciclosporin (also known as cyclosporins C, D, G and M respectively), [3-0-acetyl-MeBmt]$^1$-Ciclosporin (also known as cyclosporin A acetate), [Dihydro-MeBmt]$^1$-[Val]$^2$-Ciclosporin (also known as dihydro-cyclosporin D), [(D)Ser]$^8$-Ciclosporin, [MeIle]$^{11}$-Ciclosporin, [(D)MeVal]$^{11}$-Ciclosporin (also known as cyclosporin H), [MeAla]$^6$-Ciclosporin, [(D)Pro]$^3$-Ciclosporin and so on.

In accordance with conventional nomenclature for cyclosporins, these are defined throughout the present specification and claims by reference to the structure of Ciclosporin (i.e. Cyclosporin A). This is done by first indicating those residues in the molecule which differ from those present in Ciclosporin and then applying the term "Ciclosporin" to characterise the remaining residues which are identical to those present in Ciclosporin. At the same time the prefix "dihydro" is employed to designate cyclosporins wherein the MeBmt residue is hydrogenated (dihydro-MeBmt) i.e. where -x-y- in formula B is $-CH_2-CH_2-$. Thus [Thr]$^2$-Ciclosporine is the cyclosporin having the sequence shown in Formula A but in which αAbu at the 2-position is replaced by Thr, and [Dihydro-MeBmt]$^1$-[Val]$^2$-Ciclosporin is the cyclosporin having the sequence shown in Formula A but in which the MeBmt residue at position 1 is hydrogenated and αAbu at the 2-position is replaced by Val.

In addition, amino acid residues referred to by abbreviation, e.g. Ala, MeVal, αAbu etc. are, in accordance with conventional practice, to be understood as having the (L)-configuration unless otherwise indicated, e.g. as in the case of "(D)Ala". Residue abbreviations preceded by "Me" as in the case of "MeLeu", represent α-N-methylated residues. Individual residues of the cyclosporin molecule are numbered, as in the art, clockwise and starting with the residue MeBmt or dihydro-MeBmt in position 1. The same numerical sequence is employed throughout the present specification and claims.

In accordance with the present invention it has now been found that cyclosporins in which the residue at the 8-position is a hydroxy substituted (D)serine residue exhibit particularly interesting pharmaceutical properties.

The present invention thus provides a cyclosporin in which the residue at the 8-position is a residue of formula I

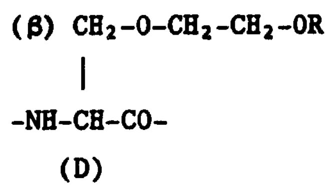

(I)

where R is hydrogen or acyl.

Preferably R is hydrogen. When R is acyl, this may represent a physiologically hydrolysable and acceptable acyl residue, that is a residue which is cleavable under physiological conditions to yield an acid (ROH) which is itself physiologically tolerable at dosages to be administered. Suitable acyl groups R include ($C_{1-4}$alkyl)carbonyl, for example acetyl, and monocyclic arylcarbonyl, for example benzoyl and salicyl.

Preferred cyclosporins in accordance with the present invention are those of formula II

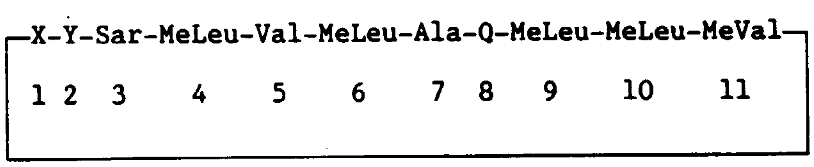

(II)

in which

X is MeBmt or dihydro-MeBmt,
Y is αAbu, Val, Thr or Nva,
and Q is a residue of formula I as described above.

The most preferred compound of the invention is [0-(2-hydroxy-ethyl)(D)Ser]$^8$-Ciclosporin, that is, the cyclosporin of Formula II in which X is MeBmt, Y is αAbu, and Q is a residue of formula I in which R is hydrogen.

The present invention also provides a process for the production of cyclosporins of the present invention, e.g. for the production of cyclosporins of formula II, which process comprises:

i) for the production of a cyclosporin (e.g. of formula II) wherein the residue at the 8-position (Q in formula II) is a residue of formula I as defined above in which R is hydrogen, reducing a corresponding cyclosporin wherein the

residue at the 8-position is a residue of formula III

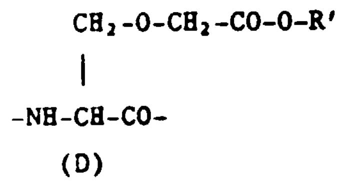

(III)

where R′ is $C_{1\text{-}3}$ alkyl, for example reducing a cyclosporin of formula IV

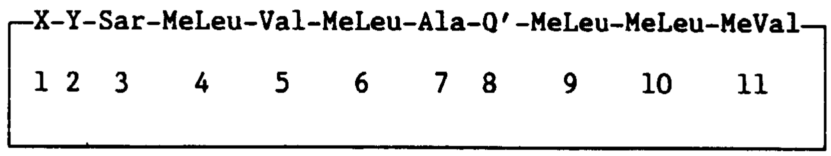

(IV)

wherein X and Y have the meanings given for formula II and Q′ is a residue of formula III as defined above; or

ii) for the production of a cyclosporin, e.g. of formula II, in which the residue at the 8-position (Q in formula II) is a residue of formula I as defined above in which R is acyl, esterifying the corresponding cyclosporin in which R is hydrogen.

Reduction in accordance with process variant i) may be carried out employing conventional methods for reducing ester groups to hydroxymethyl groups, for example by treatment with lithium borohydride under an inert atmosphere, in an appropriate solvent or diluent at ambient or moderately elevated temperature. The lithium borohydride may conveniently be replaced by sodium borohydride in the presence of a lithium halide.

Esterification in accordance with process variant ii) may be carried out in accordance with conventional procedures, for example by reaction with acid halides or anhydrides, e.g ($C_{1\text{-}4}$alkyl)carbonyl halides, in the presence of an acid binding agent.

The starting materials for process variant i) may be prepared in accordance with the following reaction method (for convenience, only the transformed residue at the 8-position is indicated):

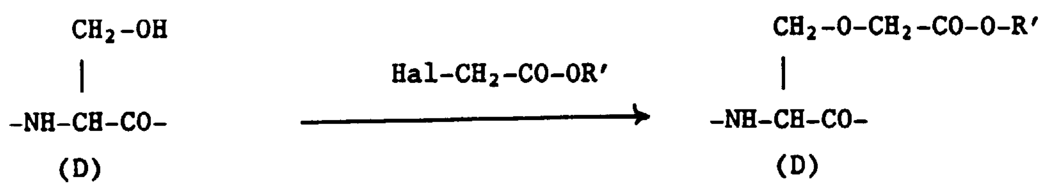


(V) . (VI) (III)

in which R′ is as defined above, and is preferably $C_3$ alkyl, particularly isopropyl; and Hal is bromine or iodine. The reaction is suitably performed in accordance with or analogously to the general procedures of Example 1 below.

Cyclosporins in which the residue at the 8-position is of formula V (that is, (D)Ser), for example [(D)Ser]$^8$-Ciclosporin, are known and are described together with processes for their production e.g. in European Patent Publication 56 782 A and British Patent Specification 2 155 936 A.

Cyclosporins according to the invention may exist in the solid state in amorphous form or in one or more crystalline forms. For example, the compound of Example 2 below exists in amorphous form or in at least three crystalline forms, distinguishable by X-ray diffraction and designated the A, B and C modifications. Of these, the B form, a hydrate obtainable by recrystallization from acetone/water, is the most stable and therefore the preferred form.

Cyclosporins in which the residue at the 8-position is a residue of formula III as defined above are new and also

form part of the present invention.

The following examples, in which all temperatures are in degrees centigrade, illustrate the invention.

**EXAMPLE 1**

[O-(isopropyloxycarbonylmethyl)(D)Ser]$^8$-Ciclosporin

[(D)Ser]$^8$-Ciclosporin (21.89 g of 88.7% pure material from a chromatography fraction of a fermentation broth, = 19.42 g, 15.93 mMol, of 100% material) is dissolved in toluene (160 ml) and tetra-N-butylammonium bromide (1.03 g, 3.2 mMol) is added. To the solution is added bromoacetic acid isopropyl ester (14.41 g, 79.7 mMol) over 5 min. at 20-25°, and the mixture is stirred for 30 min. at 20-25° while 30% aqueous sodium hydroxide solution (53.1 g, = 15.93 g pure NaOH, 398 mMol) is added dropwise. After stirring for a further 30 min. at 20-25°, the aqueous phase is separated and extracted with toluene (63 ml). The toluene phases are combined and washed with saturated aqueous ammonium chloride solution (80 ml) then with water (48 ml), then dried over aluminium oxide, filtered and evaporated to give a solid yellow foam (22-32 g).

The residue is taken up in toluene (31 ml), and the solution warmed to 65-68°. To the warm solution is added dropwise over 90 min. 125 ml n-hexane, and the mixture is cooled to 20-25° over 4 hr, stirred 10 hr at room temperature and finally cooled over 3 hr to 0-3° and stirred at this temperature for 2 hr. The resulting crystals are filtered off, washed with ice-cold toluene/hexane 1:4 (42 ml) and dried at 60° under vacuum to give the title product (19.2 g) as beige crystals of purity 87.9% (by high-pressure liquid chromatography, HPLC) which can be used in the next step without further purification.

Alternatively, further recrystallisation gives the pure title product as white crystals, m.p. 168-170°; $[\alpha]_D^{20}$ = -162.4° (methanol, c = 0.37).

**EXAMPLE 2**

[0-(2-hydroxyethyl)(D)Ser]$^8$-Ciclosporin

Step a): Crude Product

The crude product of Example 1 (14.1 g 87.9% purity = 12.39 g, 9.4 mMol, of pure material) is dissolved in absolute ethanol (315 ml) and warmed to 30-35° under a nitrogen atmosphere. To the solution is added in 7 portions over 6 hr lithium borohydride (total of 1.29 g 95% purity = 1.225 g, 56.3 mMol, 6 equivalents, of pure material). After each addition a slightly exothermic reaction occurs, with evolution of gas. The resulting beige suspension is stirred for 14 hr at 30-35°, then cooled to 20-25° and treated with 23 ml of a 20% aqueous solution of acetic acid. The clear pale yellow solution (pH 5-6) is stirred for 30 min. then evaporated to give approx. 30 ml of an oily residue.

The residue is partitioned between isopropyl acetate (IPA) (130 ml) and water (80 ml) and the aqueous phase re-extracted with IPA (40 ml). The combined IPA layers are washed with 25 ml saturated aqueous sodium bicarbonate solution, then with 30 ml water, and the solvent evaporated to give the crude title product as a beige foam (13.44 g, purity 81.8% by HPLC).

Step b): Purification

The crude product of step a) (36.2 g) is dissolved in methyl isobutyl ketone (MIBK) saturated with water, and chromatographed on Kieselgel 0.04-0.063 mm (885 g), column dimensions 460 x 70 mm diameter, using water-saturated MIBK as eluant. After an initial run-off of 2 lit., fractions containing material of >95% purity as measured by HPLC are collected giving approx. 11.5 lit. of solution, which is evaporated to dryness under vacuum to give 24.3 g of the amorphous title product as a beige foam; purity by HPLC = 95.2%.

The solid product is dissolved in acetone (40 ml) and the pale yellow solution filtered through a sintered glass funnel which is then rinsed with acetone (21 ml). To the combined acetone solution water (61 ml) is added dropwise over 90 min., with the appearance of crystals. The fine suspension is stirred for 16 hr at room temperature then cooled to 0-3° over 1 hr, stirred for a further 4 hr then filtered, washed with ice-cold acetone/water 1:1, and the damp crystals dried 14 hr at 60° under partial vacuum to give the pure title product (20.76 g) as white crystals of the B modification, purity by HPLC 98%, m.p. approx. 130° (dec.).

Further recrystallisations from acetone/water give material of even higher purity.

From the amorphous material obtained as described above, crystal modification A is obtained by recrystallization from polyethylene-glycol/water; and crystal modification C is obtained by recrystallization from ethanol/water or by ultrasonic treatment of a suspension of the amorphous form in ethanol/water. Melting points: modification A: 148°C

approx.; modification C: 120°C approx..

**EXAMPLE 3**

[O-(2-Acetyloxyethyl)(D)Ser]$^8$-Ciclosporin

The product of step a) of Example 2 (600 mg, 0.47 mMol) is dissolved in pyridine (30 ml) and mixed with acetic anhydride (30 ml). The mixture is kept at room temperature for 2.5 hr., after which the pyridine and excess acetic anhydride are evaporated under reduced pressure and the residue chromatographed on silica gel with ether/ethyl acetate 4:1 as eluent to yield the title product (430 mg). $[\alpha]_D^{20}$ = -175.8 (c= 0.25 in methanol).

Cyclosporins comprising a residue of formula I as hereinbefore defined at the 8-position (hereinafter "Product Cyclosporins"), in particular cyclosporins of formula II as hereinbefore defined, possess pharmaceutical utility as may be demonstrated, for example, in the following test methods:

1. Localised Graft-versus-Host (GvH) Reaction in the rat [Ford et al., TRANSPL. PROC. 10 (1970) 258].

Spleen cells (1 x $10^7$) from 6 week old female Wistar/Furth (WF) rats are injected subcutaneously on day 0 into the left hind-paw of female (F344 x WF)$F_1$ rats weighing about 100g. Animals are treated for 4 consecutive days and the popliteal lymph nodes are removed and weighed on day 7. The difference in weight betwen the two lymph nodes is taken as the parameter for evaluating the reation.

Product Cyclosporins inhibit GvH reaction in the above test method on administration 4x at dosages of from about 10 to about 30 mg/kg p.o., or about 1.5 to 5 mg/kg s.c..

2. Kidney Allograft Reaction in the Rat

One kidney from a female Fisher 344 rat is transplanted onto the renal vessel of a unilaterally (left side) nephrectomised WF recipient rat using an end-to-end anastomosis. Ureteric anastamosis is also end-to-end. Treatment commences on the day of transplantation and is continued for 14 days. A contralateral nephrectomy is done seven days after transplantation, leaving the recipient relying on the performance of the donor kidney. Survival of the graft is taken as the parameter for a functional graft.

Product cyclosporins are effective in maintaining graft survival in the above test method on administration at dosages of from about 2 to about 5mg/kg/day p.o..

3. Experimentally Induced Allergic Encephalomyelitis (EAE) in the Rat [Levine et al., AM. J. PATH. 47 (1965) 61; McFarlin et al, J. IMMUNOL. 113 (1974) 712; Borel, TRANSPLANT & CLIN. IMMUNOL. 13 (1981) 3].

Male Wistar rats are injected in the hind paws with a mixture of bovine spinal cord and complete Freund's adjuvant. Symptoms of the disease (paralysis of the tail and both hind legs) usually develop within 16 days. The number of diseased animals as well as the time of onset of the disease are recorded.

On administration at dosages of from about 12.5 to about 25mg/kg/day p.o.. Product Cyclosporins inhibit disease onset in the above test model.

4. Freund's Adjuvant Arthritis [Winter & Nuss, ARTHRITIS AND RHEUMATISM 9 (1966) 394; Billingham & Davies, HANDBOOK OF EXPERIMENTAL PHARMACOL (Vane & Ferreira Eds, Springer-Verlag, Berlin,) 50/II, (1979) 108-144]

OFA and Wistar rats (male or female, 150g body weight) are injected i.c. at the base of the tail or in the hind paw with 0.1 ml of mineral oil containing 0.6 mg of lyophilised heat-killed Mycobacterium smegmatis. In the developing arthritis model, treatment is started immediately after the injection of the adjuvant (days 1 - 18); in the established arthritis model treatment is started on day 14, when the secondary inflammation is well developed (days 14-20). At the end of the experiment, the swelling of the joints is measured by means of a micro-caliper.

Product Cyclosporins are effective in preventing or inhibiting disease progression in both developing and established test models on administration at dosages of from about 3 to about 20mg/kg/day p.o..

Product Cyclosporins also exhibit favourable toxicity, e.g. hepatotoxic and/or nephrotoxic, profiles as compared with known cyclosporins, for example Ciclosporin, as may be demonstrated by determination of their influence on relevant parameters, e.g. blood creatinine; glomerular filtration rate; urea, bilirubin and bile acid levels, following long term administration to rats at immunosuppressively effective dosage rates. Product Cyclosporins are thus characterised by an improved therapeutic ratio as compared with known cyclosporins.

Product Cyclosporins are therefore useful as pharmaceuticals, e.g. as immunosuppressive as well as anti-inflammatory agents.

Product Cyclosporins are in particular useful for the prevention of organ or tissue transplant rejection, e.g. for the treatment of recipients of heart, lung, combined heart-lung, liver, kidney, pancreatic, skin or corneal transplants. They are also indicated for the prevention of graft-versus-host disease, such as following bone marrow transplants.

Product Cyclosporins are also useful for the treatment of autoimmune disease and of inflammatory conditions, in particular inflammatory conditions with an aetiology including an autoimmune component such as arthritis (for example rheumatoid arthritis, arthritis chronica progrediente and arthritis deformans) and rheumatic diseases. Specific autoimmune diseases for which product Cyclosporins may be employed include autoimmune haematological disorders (including e.g. haemolytic anaemia, aplastic anaemia, pure red cell anaemia and idiopathic thrombocytopenia), systemic lupus erythematosus, polychondritis, sclerodoma, Wegener granulamatosis, dermatomyositis, chronic active hepatitis, myasthenia gravis, psoriasis, Steven-Johnson syndrome, idiopathic sprue, autoimmune inflammatory bowel disease (including e.g. ulcerative colitis and Crohn's disease) endocrine ophthalmopathy, Graves disease, sarcoidosis, multiple sclerosis, primary billiary cirrhosis, juvenile diabetes (diabetes mellitus type I), uveitis (anterior and posterior), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minimal change nephropathy).

Product Cyclosporins are further indicated for use in the treatment of other diseases or conditions for which cyclosporin, e.g. Ciclosporin, therapy is practiced or proposed, for example, for the treatment of alopecia/the promotion of hair growth and for the treatment of asthma, e.g. on administration by inhalation. They are also indicated for use as anti-parasitic agents, in particular for the treatment of parasitic, e.g. protozoal, fungal or vermicular infection or invasion, for example in the treatment of filariasis, schistosomiasis, coccidiomycosis or plasmodial infection, e.g. malaria. They are yet further indicated for use in the reversal of induced resistance of malignancies to other chemotherapy, as well as the enhancement of wound healing.

For the above indications the appropriate dosage will, of course, vary depending, for example, on the particular Product Cyclosporin employed, the subject to be treated, the mode of administration and the nature and severity of the condition being treated. However, in general, satisfactory results in animals are obtained at daily dosages of from about 1 to about 10mg/kg/day p.o.. In larger mammals, for example humans, an indicated daily dosage is in the range of from about 50 to about 800mg of Product Cyclosporin administered orally once or, more suitably, in divided dosages two to four times/day.

In organ transplantation in humans, an initial single oral dose of 10-15 mg/kg Product Cyclosporin 4-12 hours prior to surgery is indicated. This daily dose is maintained for one to two weeks post-operatively, before being gradually reduced in accordance with blood levels until a maintenance dose of about 2-6 mg/kg/day is reached. When Product Cyclosporins are given along with other immunosuppressants (e.g. with corticosteroids as part of a triple or quadruple drug therapy) lower doses (e.g. 1 mg/kg/day i.v.; 6 mg/kg/day oral initially) may be used.

Product Cyclosporins may be administered by any conventional route, in particular enterally, e.g. orally, for example in the form of solutions for drinking, tablets or capsules or parenterally, for example in the form of injectible solutions or suspensions. Normally for systemic administration oral dosage forms are preferred, although for some conditions, for example for prevention of rejection of liver transplants, an intravenously injectable form is desirable. Product Cyclosporins may also be administered topically or dermally, e.g. in the form of a dermal cream or gel or like preparation or, for the purposes of application to the eye, in the form of an occular cream, gel or eye-drop preparation. Suitable unit dosage forms for oral administration comprise e.g. from 25 to 200mg Product Cyclosporin per dosage.

Preferred galenic formulations for Product Cyclosporins include those based on microemulsions as described in British Patent Application 2 222 770A, which include topical as well as oral forms; also oral and injectable forms obtained from solid solutions comprising a fatty acid saccharide monoester, e.g. saccharose monolaurate, as described in British Patent Application 2 209 671A.

Examples of particularly preferred formulations suitable for filling soft gelatine capsules for oral administration include:

| Formulation Example A: | |
|---|---|
| Product of Ex.2 | 50.0 mg |
| Glycofurol 75 | 180.0 mg |
| Miglyol 812 | 90.0 mg |
| Cremophor RH40 | 180.0 mg |
| alpha-Tocopherol | 0.5 mg |

| Formulation Example B: | |
|---|---|
| Product of Ex.2 | 100.0 mg |
| Tetraglycol | 20.0 mg |
| Captex 800 | 20.0 mg |
| Nikkol HC0-40 | 860.0 mg |
| Butylhydroxytoluene(BHT) | 1.0 mg |

| Formulation Example C: | |
|---|---|
| Product of Ex.2 | 25.0 mg |
| Glycofurol 75 | 100.0 mg |
| Miglyol 812 | 35.0 mg |
| Cremophor RH 40 | 90.0 mg |
| Butylhydroxyanisole(BHA) | 0.2 mg |

| Formulation Example D: | |
|---|---|
| Product of Ex.2 | 10.0 mg |
| Tetraglycol | 10.0 mg |
| Myritol | 5.0 mg |
| Cremophor RH 40 | 75.0 mg |
| alpha-Tocopherol | 0.1 mg |

The individual components of these formulations, as well as.the methods for their preparation, are fully described in British Patent Application 2 222 770, the contents of which are incorporated herein by reference.

Determined ED values in the above described Test Methods 1 to 4 for the cyclosporin of Example 2 are as follows:

| TEST METHOD | ED mg/kg | = Dosage required... |
|---|---|---|
| 1 | 25 (ED50) | ... to reduce average difference in weight between node pairs by 50% as compared with untreated controls. |
| 2 | 2.5 | ... to prolong survival to >100 days in 100% of treated rats. |
| 3 | 25 | ... to reduce disease symptomatology by 100% as compared with untreated controls. |
| 4 | 20 | ... to reduce swelling to that recorded for untreated controls. |

In accordance with the foregoing the present invention also provides in a further series of embodiments:
A pharmaceutical composition for use in one of the methods (A) or (B):

A. A method of effecting immunosuppression in a subject in need of such treatment which method comprises administering to said subject an effective amount of a Product Cyclosporin.

B. A method:

1) for the prevention of organ transplant rejection, for example for the treatment of recipients of organ transplants of any of the particular types listed above; or

2) for the prevention of graft-versus-host disease, for example in recipients of bone marrow transplants; or

3) for the treatment of autoimmune disease or for the treatment of inflammatory conditions, for example for the treatment of any such disease or condition listed above; or

4) for the treatment of alopecia or for the promotion of hair growth; or

5) for the treatment of parasitic infection or invasion, for example, for the treatment of protozoal, fungal or vermicular infection or invasion, e.g. for the treatment of coccidiomycosis or malaria; or

6) for the reversal of reduced resistance of malignancies to chemotherapy, for the enhancement of wound healing or for the treatment of asthma;

in a subject in need of such treatment, which method comprises administering to said subject an effective amount of a Product Cyclosporin.

C. A Product Cyclosporin for use as a pharmaceutical, e.g. for use as an immunosuppressant or for use in the treatment of any disease or condition as set forth under B above.

D. A pharmaceutical composition comprising a Product Cyclosporin in association with a pharmaceutically acceptable diluent or carrier.

## Claims

**Claims for the following Contracting States : AT, BE, CH, DE, DK, FR, GB, IT, LI, LU, NL, SE**

**1.** A cyclosporin wherein the residue at the 8-position is a residue of formula I

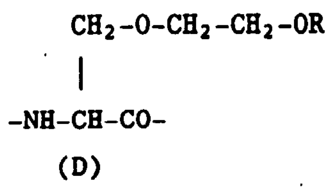

(I)

wherein R is hydrogen or a physiologically hydrolysable and acceptable acyl residue.

**2.** A cyclosporin according to claim 1 of formula II

**X-Y-Sar-MeLeu-Val-MeLeu-Ala-Q-MeLeu-MeLeu-MeVal**
**1 2 3 4 5 6 7 8 9 10 11**

**(II)**

in which

X is MeBmt or dihydro-MeBmt,
Y is αAbu, Val, Thr or Nva and
Q is a residue of formula I as defined in claim 1.

**3.** A cyclosporin according to claim 1 or claim 2 in which R is hydrogen.

**4.** [0-(2-Hydroxyethyl)(D)Ser]$^8$-Ciclosporin.

**5.** A cyclosporin according to claim 1 or claim 2 in which R is acetyl.

**6.** [0-(2-Acetyloxyethyl)(D)Ser]$^8$-Ciclosporin

**7.** A process for the production of a cyclosporin according to claim 1 which process comprises:

i) for the production of a cyclosporin in which the residue at the 8-position is a residue of formula I in which R is hydrogen, reducing a corresponding cyclosporin wherein the residue at the 8-position is a residue of formula III

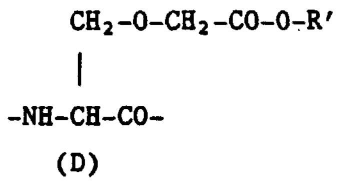

(III)

where R′ is $C_{1-3}$ alkyl, or

ii) for the production of a cyclosporin in which the residue at the 8-position is a residue of formula I in which R is acyl, esterifying the corresponding cyclosporin in which R is hydrogen.

**8.** A cyclosporin in which the residue at the 8-position is a residue of formula III

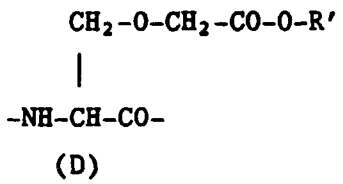

(III)

where R′ is $C_{1-3}$ alkyl.

**9.** [O-(Isopropyloxycarbonylmethyl)(D)Ser]$^{8}$-Ciclosporin.

**10.** A cyclosporin according to any one of claims 1 to 6 for use as a pharmaceutical.

**11.** A pharmaceutical composition comprising a cyclosporin according to any one of claims 1 to 6 together with a pharmaceutically acceptable diluent or carrier.

**Claims for the following Contracting States : ES, GR**

**1.** A process for the production of a cyclosporin wherein the residue at the 8-position is a residue of formula I

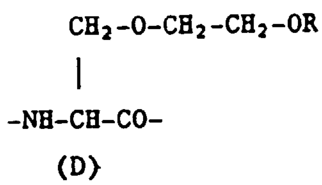

(I)

wherein R is hydrogen or a physiologically hydrolysable and acceptable acyl residue, which process comprises:

i) for the production of a cyclosporin in which the residue at the 8-position is a residue of formula I in which R is hydrogen, reducing a corresponding cyclosporin wherein the residue at the 8-position is a residue of formula III

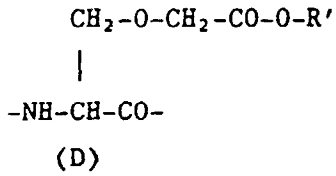

(III)

where R′ is $C_{1-3}$ alkyl, or

ii) for the production of a cyclosporin in which the residue at the 8-position is a residue of formula I in which R is acyl, esterifying the corresponding cyclosporin in which R is hydrogen.

**2.** A process according to claim 1 for the production of a cyclosporin of formula II

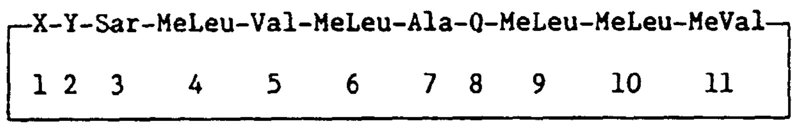

(II)

in which

X is MeBmt or dihydro-MeBmt,
Y is αAbu, Val, Thr or Nva and
Q is a residue of formula I as defined in claim 1.

**3.** A process according to variant i) of claim 1 for the production of a cyclosporin as defined in claim 2 in which R is hydrogen.

**4.** A process for the production of [O-(2-hydroxyethyl)(D)Ser]$^8$-Ciclosporin comprising the step of reducing [O-(isopropyloxycarbonylmethyl)(D)Ser]$^8$-Ciclosporin.

**5.** A process according to variant ii) of claim 1 for the production of a cyclosporin as defined in claim 2 in which R is acetyl.

**6.** A process for the preparation of [O-(2-acetyloxyethyl)(D)Ser]$^8$-Ciclosporin comprising the step of acetylating [O-(2-hydroxyethyl) (D)Ser]$^8$-Ciclosporin.

**7.** A process for the production of a cyclosporin in which the residue at the 8-position is a residue of formula III

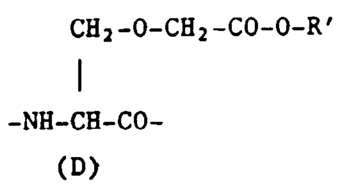

(III)

where R′ is $C_{1-3}$ alkyl, comprising the step of reacting a cyclosporin in which the residue at the 8-position is a residue of formula (V)

```
   CH2-OH
   |
-NH-CH-CO-          (V)
   (D)
```

with a compound of formula Hal-$CH_2$-CO-OR′ where R′ is as defined above and Hal is bromine or iodine.

**8.** A process for the preparation of [O-(Isopropyloxycarbonylmethyl) (D)Ser]$^8$-Ciclosporin, comprising the step of reacting [(D)Ser]$^8$-Ciclosporin with isopropyl bromoacetate.

**9.** A pharmaceutical composition comprising a cyclosporin as defined in any one of claims 1 to 6 together with a pharmaceutically acceptable diluent or carrier.

**Patentansprüche**

**Patentansprüche für folgende Vertragsstaaten : AT, BE, CH, DE, DK, FR, GB, IT, LI, LU, NL, SE**

**1.** Cyclosporin, bei dem der Rest an Position 8 ein Rest der Formel I ist

```
   CH2-O-CH2-CH2-OR
   |
-NH-CH-CO-                 (I)
   (D)
```

worin R Wasserstoff oder ein physiologisch hydrolysierbarer und annehmbarer Acylrest ist.

**2.** Cyclosporin nach Anspruch 1 der Formel II

```
┌X-Y-Sar-MeLeu-Val-MeLeu-Ala-Q-MeLeu-MeLeu-MeVal┐
│1 2  3    4    5    6    7  8   9     10    11  │   (II)
└───────────────────────────────────────────────┘
```

worin

X MeBmt oder Dihydro-MeBmt ist,
Y αAbu, Val, Thr oder Nva ist und
Q ein Rest der Formel I, wie in Anspruch 1 definiert, ist.

**3.** Cyclosporin nach Anspruch 1 oder Anspruch 2, worin R Wasserstoff ist.

**4.** [O-(2-Hydroxyethyl)(D)Ser]$^8$-Ciclosporin.

**5.** Cyclosporin nach Anspruch 1 oder Anspruch 2, worin R ein Acetylrest ist.

**6.** [O-(2-Acetyloxyethyl)(D)Ser]$^8$-Ciclosporin.

**7.** Verfahren zur Herstellung eines Cyclosporins nach Anspruch 1, wobei das Verfahren umfaßt:

i) zur Herstellung eines Cyclosporins, bei dem der Rest an Position 8 ein Rest der Formel I ist, worin R Wasserstoff ist, ipdem man ein entsprechendes Cyclosporin, worin der Rest an Position 8 ein Rest der Formel III ist

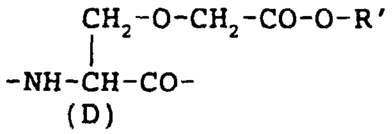

(III)

worin R' ein $C_{1-3}$-Alkylrest ist, reduziert oder

ii) zur Herstellung eines Cyclosporins, worin der Rest an Position 8 ein Rest der Formel I ist, worin R ein Acylrest ist, das entsprechende Cyclosporin, worin R Wasserstoff ist, verestert.

**8.** Cyclosporin, bei dem der Rest an Position 8 ein Rest der Formel III ist

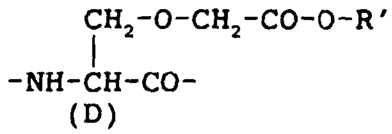

(III)

worin R' ein $C_{1-3}$-Alkylrest ist.

**9.** [O-(Isopropyloxycarbonylmethyl)(D)Ser]$^{8}$-Ciclosporin.

**10.** Cyclosporin nach einem der Ansprüche 1 bis 6 zur Verwendung als Pharmazeutikum.

**11.** Pharmazeutische Zusammensetzung enthaltend ein Cyclosporin nach einem der Ansprüche 1 bis 6 zusammen mit einem pharmazeutisch annehmbaren Verdünnungsmittel oder Träger.

**Patentansprüche für folgende Vertragsstaaten : ES, GR**

**1.** Verfahren zur Herstellung eines Cyclosporins, worin der Rest an Position 8 ein Rest der Formel I ist

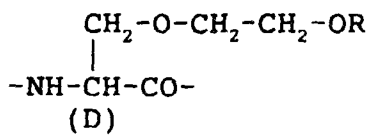

(I)

worin R Wasserstoff oder ein physiologisch hydrolysierbarer und annehmbarer Acylrest ist, wobei das Verfahren umfaßt:

i) zur Herstellung eines Cyclosporins, bei dem der Rest an Position 8 ein Rest der Formel I ist, worin R Wasserstoff ist, indem man ein entsprechendes Cyclosporin, worin der Rest an Position 8 ein Rest der Formel III ist

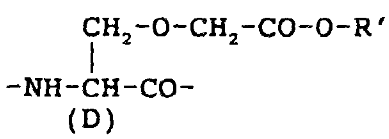

(III)

worin R' ein $C_{1-3}$-Alkylrest ist, reduziert oder

ii) zur Herstellung eines Cyclosporins, worin der Rest an Position 8 ein Rest der Formel I ist, worin R ein Acylrest ist, das entsprechende Cyclosporin, worin R Wasserstoff ist, verestert.

**2.** Verfahren nach Anspruch 1 zur Herstellung eines Cyclosporins der Formel II

X-Y-Sar-MeLeu-Val-MeLeu-Ala-Q-MeLeu-MeLeu-MeVal
1 2 3 4 5 6 7 8 9 10 11 (II)

worin

X MeBmt oder Dihydro-MeBmt ist,
Y αAbu, Val, Thr oder Nva ist und
Q ein Rest der Formel I, wie in Anspruch 1 definiert, ist.

**3.** Verfahren gemäß Variante i) von Anspruch 1 zur Herstellung eines Cyclosporins, wie in Anspruch 2 definiert, worin R Wasserstoff ist.

**4.** Verfahren zur Herstellung von [O-(2-Hydroxyethyl)(D)Ser]$^8$-Ciclosporin umfassend die Stufe, daß man [O-(Isopropyloxycarbonylmethyl)(D)Ser]$^8$-Ciclosporin reduziert.

**5.** Verfahren nach Variante ii) von Anspruch 1 zur Herstellung eines Cyclosporins, wie in Anspruch 2 definiert, worin R ein Acetylrest ist.

**6.** Verfahren zur Herstellung von [O-(2-Acetyloxyethyl)(D)Ser]$^8$-Ciclosporin umfassend die Stufe, daß man [O-(2-Hydroxyethyl)(D)-Ser]$^8$-Ciclosporin acetyliert.

**7.** Verfahren zur Herstellung eines Cyclosporins, bei dem der Rest an Position 8 ein Rest der Formel III ist

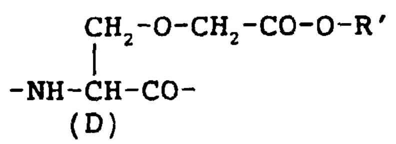

(III)

worin R' ein $C_{1-3}$-Alkylrest ist, umfassend die Stufe, daß man ein Cyclosporin, in dem der Rest an Position 8 ein Rest der Formel V ist

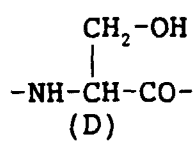

(V)

mit einer Verbindung der Formel Hal-$CH_2$-CO-OR', worin R' wie oben definiert ist und Hal Brom oder Iod ist, umsetzt.

**8.** Verfahren zur Herstellung von [O-(Isopropyloxycarbonylmethyl)(D)Ser]$^8$-Ciclosporin umfassen die Stufe, daß man [(D)Ser]$^8$-Ciclosporin mit Isopropylbromacetat umsetzt.

**9.** Pharmazeutische Zusammensetzung enthaltend ein Cyclosporin nach einem der Ansprüche 1 bis 6 zusammen mit einem pharmazeutisch annehmbaren Verdünnungsmittel oder Träger.

## Revendications

**Revendications pour les Etats contractants suivants : AT, BE, CH, DE, DK, FR, GB, IT, LI, LU, NL, SE**

**1.** Une cyclosporine dans laquelle le résidu en position 8 est un groupe de formule I

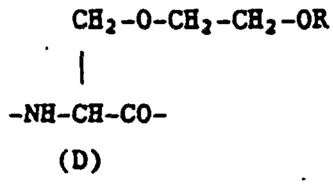

(I)

où R signifie l'hydrogène ou un reste acyle physiologiquement hydrolysable et acceptable.

**2.** Une cyclosporine selon la revendication 1 de formule II

X-Y-Sar-MeLeu-Val-MeLeu-Ala-Q-MeLeu-MeLeu-MeVal
1 2 3 4 5 6 7 8 9 10 11 (II)

dans laquelle

X signifie MeBmt ou dihydro-MeBmt,
Y signifie aAbu, Val, Thr ou Nva, et
Q signifie un groupe de formule I tel que défini à la revendication 1.

**3.** Une cyclosporine selon la revendication 1 ou 2, dans laquelle R signifie l'hydrogène.

**4.** La [O-(2-hydroxyéthyl)(D)Ser]$^8$-Ciclosporine.

**5.** Une cyclosporine selon la revendication 1 ou 2, dans laquelle R signifie un groupe acétyle.

**6.** La [O-(2-acétyloxyéthyl)(D)Ser]$^8$-Ciclosporine.

**7.** Un procédé de préparation d'une cyclosporine selon la revendication 1, procédé selon lequel:

i) pour préparer une cyclosporine dans laquelle le résidu en position 8 est un groupe de formule I où R signifie l'hydrogène, on réduit une cyclosporine correspondante dans laquelle le résidu en position 8 est un groupe de formule III

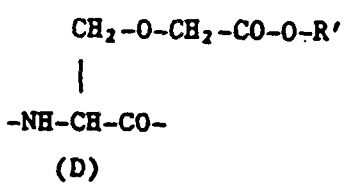

(III)

où R' signifie un groupe alkyle en $C_1$-$C_3$, ou bien
ii) pour préparer une cyclosporine dans laquelle le résidu en position 8 est un groupe de formule I où R signifie un groupe acyle, on estérifie la cyclosporine correspondante dans laquelle R signifie l'hydrogène.

**8.** Une cyclosporine dans laquelle le résidu en position 8 est un groupe de formule III

```
     CH2-O-CH2-CO-O-R'            (III)
     |
-NH-CH-CO-
    (D)
```

où R' signifie un groupe alkyle en $C_1$-$C_3$.

**9.** La [O-(isopropyloxycarbonylméthyl)(D)Ser]$^8$-Ciclosporine.

**10.** Une cyclosporine selon l'une quelconque des revendications 1 à 6, pour une utilisation comme médicament.

**11.** Une composition pharmaceutique comprenant une cyclosporine selon l'une quelconque des revendications 1 à 6, en association avec un diluant ou véhicule pharmaceutiquement acceptable.

**Revendications pour les Etats contractants suivants : ES, GR**

**1.** Un procédé de préparation d'une cyclosporine dans laquelle le résidu en position 8 est un groupe de formule I

```
     CH2-O-CH2-CH2-OR
     |                            (I)
-NH-CH-CO-
    (D)
```

où R signifie l'hydrogène ou un reste acyle physiologiquement hydrolysable et acceptable, procédé selon lequel:

i) pour préparer une cyclosporine dans laquelle le résidu en position 8 est un groupe de formule I où R signifie l'hydrogène, on réduit une cyclosporine correspondante dans laquelle le résidu en position 8 est un groupe de formule III

```
     CH2-O-CH2-CO-O-R'            (III)
     |
-NH-CH-CO-
    (D)
```

où R' signifie un groupe alkyle en $C_1$-$C_3$, ou bien

ii) pour préparer une cyclosporine dans laquelle le résidu en position 8 est un groupe de formule I où R signifie un groupe acyle, on estérifie la cyclosporine correspondante dans laquelle R signifie l'hydrogène.

**2.** Un procédé selon la revendication 1, pour la préparation d'une cyclosporine de formule II

```
┌X-Y-Sar-MeLeu-Val-MeLeu-Ala-Q-MeLeu-MeLeu-MeVal┐
│1 2  3    4    5    6    7  8   9     10    11 │   (II)
└───────────────────────────────────────────────┘
```

dans laquelle

X signifie MeBmt ou dihydro-MeBmt,
Y signifie αAbu, Val, Thr ou Nva, et
Q signifie un groupe de formule I tel que défini à la revendication 1.

**3.** Un procédé selon la variante i) de la revendication 1, pour la préparation d'une cyclosporine telle que définie à la revendication 2, dans laquelle R signifie l'hydrogène.

**4.** Un procédé de préparation de la [O-(2-hydroxyéthyl)(D)Ser]$^8$-Ciclosporine, comprenant l'étape d'une réduction de la [O-(isopropyloxycarbonylméthyl)(D)Ser]$^8$-Ciclosporine.

**5.** Un procédé selon la variante ii) de la revendication 1, pour la préparation d'une cyclosporine telle que définie à la revendication 2, dans laquelle R signifie un groupe acétyle.

**6.** Un procédé de préparation de la [O-(2-acétyloxyéthyl)(D)Ser]$^8$-Ciclosporine, comprenant l'étape d'une acétylation de la [O-(2-hydroxyéthyl)-(D)Ser]$^8$-Ciclosporine.

**7.** Un procédé de préparation d'une cyclosporine dans laquelle le résidu en position 8 est un groupe de formule III

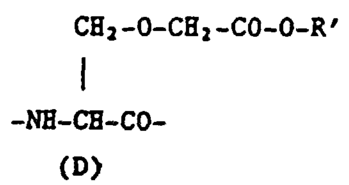

(III)

où R' signifie un groupe alkyle en $C_{1-3}$, comprenant l'étape d'une réaction d'une cyclosporine dans laquelle le résidu en position 8 est un groupe de formule (V)

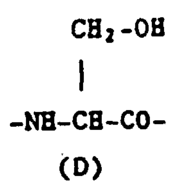

(V)

avec un composé de formule Hal-$CH_2$-CO-OR' où R' est tel que défini plus haut et Hal signifie le brome ou l'iode.

**8.** Un procédé de préparation de la [O-(isopropyloxycarbonylméthyl)(D)Ser]$^8$-Ciclosporine, comprenant l'étape d'une réaction de la [(D)Ser]$^8$-Ciclosporine avec le bromoacétate d'isopropyle.

**9.** Une composition pharmaceutique comprenant une cyclosporine selon l'une quelconque des revendications 1 à 6, en association avec un diluant ou véhicule pharmaceutiquement acceptable.